# EUROPEAN PATENT APPLICATION

(11) **EP 4 772 267 A1**
(43) Date of publication of application: **08.07.2026**
(21) Application number: 24889966.8
(22) Date of filing: 10.04.2024
(51) Int. Cl.: B01J 8/02, B01J 8/00, C07K 1/04, C07K 1/06

(54) **POLYPEPTIDE SOLID-PHASE SYNTHESIS DEVICE**

(30) Priority: 15.11.2023 CN 202311524387
(71) Applicant: Tianjin Asymchem Medical Science And Technology Development Co., Ltd., Tianjin 300467 (CN)
(72) Inventor: HONG, Liang, Tianjin 300467 (CN); TAO, Jian, Tianjin 300467 (CN); ZHOU, Hongru, Tianjin 300467 (CN); WANG, Junqiang, Tianjin 300467 (CN); FENG, Miao, Tianjin 300467 (CN); WANG, Bohui, Tianjin 300467 (CN)
(74) Representative: Zacco GmbH
(86) International application number: PCT/CN2024/087058
(87) International publication number: WO 2025/102592

(57) **Abstract**

Provided in the present disclosure is a solid-phase peptide synthesis apparatus. The solid-phase peptide synthesis apparatus includes a first circulation pipeline, a second circulation pipeline, a first switching valve, and a second switching valve. The first circulation pipeline includes a synthetic column, a third switching valve, and a first pump body, which are sequentially arranged from upstream to downstream. The second circulation pipeline includes a fourth switching valve, a second pump body, and an activation reactor, which are sequentially arranged from upstream to downstream. Through the technical solutions provided in the present disclosure, the problems of low synthesis efficiency, complex batch operations, and large amino acid usage amount of solid-phase peptide synthesis apparatuses in the related art can be solved.

## Description

This application claims priority to Chinese Patent Application No. 202311524387.6 filed to the China National Intellectual Property Administration on November 15, 2023 and entitled "Solid-phase Peptide Synthesis Apparatus".

### Technical Field

The present disclosure relates to the technical field of peptide synthesis apparatuses, and specifically to a solid-phase peptide synthesis apparatus.

### Background

Peptide is a class of compounds formed by linking a plurality of amino acids through peptide bonds. In recent years, with the advancement and maturation of the peptide synthesis technology, peptide drugs have become one of the hotspots in drug research and development. The peptide drugs, with their broad range of indications, high safety, and significant therapeutic efficacy, have been widely applied in the prevention, diagnosis, and treatment of diseases such as tumors, cardiovascular and cerebrovascular diseases, hepatitis, diabetes, AIDS, etc., and thus have broad development prospects.

With the rapid advancement of technologies (e.g., the emergence of solid-phase peptide synthesis), devices, processes, etc. related to peptide synthesis, the research and development costs and production costs of the peptide drugs have significantly decreased, such that the development of the peptide drugs continues to gain momentum. In the solid-phase synthesis process for peptide, a peptide synthesizer is commonly used for synthesis.

However, the synthesis process of solid-phase peptide synthesizers in the related art is batch-based, time-consuming, and labor-intensive, which often requires manual intervention, resulting in low synthesis efficiency and high amino acid consumption.

### Summary

The present disclosure provides a solid-phase peptide synthesis apparatus, so as to solve the problems of low synthesis efficiency, complex batch operations, and large amino acid usage amount of solid-phase peptide synthesis apparatuses in the related art.

The present disclosure provides a solid-phase peptide synthesis apparatus. The solid-phase peptide synthesis apparatus includes a first circulation pipeline, a second circulation pipeline, a first switching valve, and a second switching valve. The first circulation pipeline includes a synthetic column, a third switching valve, and a first pump body, which are sequentially arranged from upstream to downstream; the second circulation pipeline includes a fourth switching valve, a second pump body, and an activation reactor, which are sequentially arranged from upstream to downstream; the first switching valve is provided with a switching inlet a1, a switching outlet a1, and a switching outlet a2; the second switching valve includes a switching inlet b1, a switching inlet b2, a switching outlet b1, and a switching outlet b2; the third switching valve is provided with a sample inlet c1, a switching inlet c1, and a switching outlet c1; the fourth switching valve is provided with a sample inlet d1, a switching inlet d1, and a switching outlet d1; an outlet of the first pump body and an outlet of the second pump body both communicate with the switching inlet a1 of the first switching valve; the switching outlet a1 of the first switching valve communicates with the switching inlet b1 of the second switching valve; the switching outlet b1 of the second switching valve communicates with an inlet of the synthetic column; an outlet of the synthetic column communicates with the switching inlet c1 of the third switching valve; the switching outlet c1 of the third switching valve communicates with an inlet of the first pump body; the switching outlet a2 of the first switching valve communicates with an inlet of the activation reactor; an outlet of the activation reactor communicates with the switching inlet b2 of the second switching valve; the switching outlet b2 of the second switching valve communicates with the switching inlet d1 of the fourth switching valve; and the switching outlet d1 of the fourth switching valve communicates with an inlet of the second pump body.

Further, the first circulation pipeline further includes a fifth switching valve; the fifth switching valve is provided with a switching inlet e1, a first flushing inlet, and a switching outlet e1; the outlet of the synthetic column communicates with the switching inlet e1 of the fifth switching valve; the switching outlet e1 of the fifth switching valve communicates with the switching inlet c1 of the third switching valve; and the switching outlet b2 of the second switching valve also communicates with the first flushing inlet of the fifth switching valve.

Further, the fifth switching valve is further provided with a second flushing inlet; the first switching valve is further provided with a switching outlet a3; and the switching outlet a3 of the first switching valve communicates with the second flushing inlet of the fifth switching valve.

Further, the solid-phase peptide synthesis apparatus further includes a waste liquid pipeline. The third switching valve is further provided with a waste liquid outlet, and the waste liquid outlet of the third switching valve communicates with an inlet of the waste liquid pipeline.

Further, a flow restrictor and a waste liquid outlet valve are arranged on the waste liquid pipeline; and the waste liquid outlet valve is located downstream of the flow restrictor.

Further, the solid-phase peptide synthesis apparatus further includes a detector that is capable of detecting a synthesis parameter. The detector is disposed on the first circulation pipeline.

Further, the detector includes a UV detector, and the UV detector is located between the synthetic column and the third switching valve; and/or the detector further includes a conductivity detector, and the conductivity detector is located between the first pump body and the first switching valve.

Further, the solid-phase peptide synthesis apparatus further includes a controller. The first pump body, the second pump body, and the detector are respectively in signal connection with the controller.

Further, the solid-phase peptide synthesis apparatus further includes two first sample injection valves, where outlets of the two first sample injection valves both communicate with the sample inlet d1 of the fourth switching valve; and/or the solid-phase peptide synthesis apparatus further includes a second sample injection valve, where an outlet of the second sample injection valve communicates with the sample inlet c1 of the third switching valve.

Further, a pipe body of the first circulation pipeline is made of a Poly Ether Ether Ketone (PEEK), Fluorinated Ethylene Propylene (FEP), or stainless steel material; and/or a pipe body of the second circulation pipeline is made of the PEEK, FEP, or stainless steel material.

By using the technical solutions of the present disclosure, the solid-phase peptide synthesis apparatus includes the first circulation pipeline, the second circulation pipeline, the first switching valve, and the second switching valve. The first circulation pipeline, the second circulation pipeline, the first switching valve, and the second switching valve can constitute a deprotection circulation, an amino acid activation circulation, and an amino acid coupling circulation according to their communication relationships. In the deprotection circulation, a deprotection reagent enters the third switching valve via the sample inlet c1, is conveyed to the first switching valve via the first pump body, and then enters the synthetic column via the second switching valve; and at the moment, a circulation pipeline of synthetic column→third switching valve→first pump body→first switching valve→second switching valve→synthetic column is opened to cause the deprotection reagent to be in full contact with a carrier in the synthetic column. In the amino acid activation circulation, amino acids and a reagent are combined into a single stream via the first pump body and the second pump body, enter the first switching valve, and then enter the activation reactor; and then, a circulation pipeline of activation reactor→second switching valve→fourth switching valve→second pump body→first switching valve→activation reactor is opened to cause the amino acids to be in full contact with an activating agent, thereby achieving an effect of activating the amino acids. In the amino acid coupling circulation, the amino acids are fully activated and then enter the synthetic column; and then, a circulation pipeline of synthetic column→third switching valve→first pump body→first switching valve→second switching valve→synthetic column is opened to cause the activated amino acids to be in full contact with the carrier. By using the above structures, continuous synthesis can be realized, synthesis continuity is ensured maximally, operation time of each process and between processes is shortened, production efficiency may be improved, and the amount of the amino acids used may be reduced, thereby reducing costs.

### Brief Description of the Drawings

The drawings described herein are used to provide a further understanding of the present disclosure, and constitute a part of the present disclosure. The exemplary embodiments and descriptions of the present disclosure are used to explain the present disclosure and do not constitute an improper limitation of the present disclosure. In the drawings:

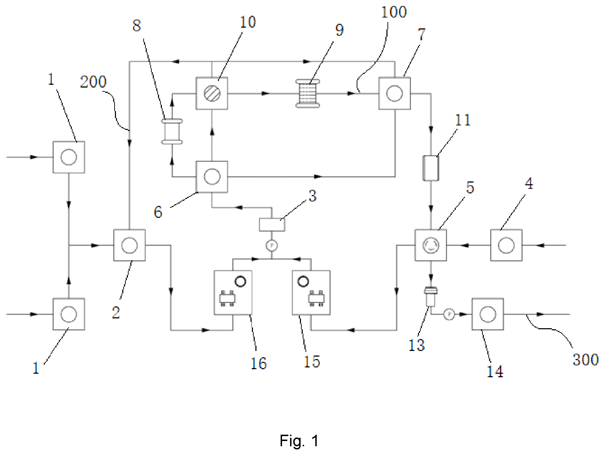

Fig. 1 is a schematic diagram of a solid-phase peptide synthesis apparatus according to an embodiment of the present disclosure.

The above drawings include the following reference numerals:
100. First circulation pipeline; 200. Second circulation pipeline; 300. Waste liquid pipeline;
1. First sample injection valve; 2. Fourth switching valve; 3. Conductivity detector; 4. Second sample injection valve; 5. Third switching valve; 6. First switching valve; 7. Fifth switching valve; 8. Activation reactor; 9. Synthetic column; 10. Second switching valve; 11. UV detector; 13. Flow restrictor; 14. Waste liquid outlet valve; 15. First pump body; 16. Second pump body.

### Detailed Description of the Embodiments

The technical solutions in the embodiments of the present disclosure will be clearly and completely described below in combination with the drawings in the embodiments of the present disclosure. It is apparent that the described embodiments are only part of the embodiments of the present disclosure, not all the embodiments. The following description of at least one exemplary embodiment is merely illustrative in nature and is never intended to limit the present disclosure and application or use thereof in any way. All other embodiments obtained by those of ordinary skill in the art on the basis of the embodiments in the present disclosure without creative work all fall within the scope of protection of the present disclosure.

As shown in Fig. 1, an embodiment of the present disclosure provides a solid-phase peptide synthesis apparatus. The solid-phase peptide synthesis apparatus includes a first circulation pipeline 100, a second circulation pipeline 200, a first switching valve 6, and a second switching valve 10. The first circulation pipeline 100 includes a synthetic column 9, a third switching valve 5, and a first pump body 15, which are sequentially arranged from upstream to downstream. The second circulation pipeline 200 includes a fourth switching valve 2, a second pump body 16, and an activation reactor 8, which are sequentially arranged from upstream to downstream. The first switching valve 6 is provided with a switching inlet a1, a switching outlet a1, and a switching outlet a2; the second switching valve 10 includes a switching inlet b1, a switching inlet b2, a switching outlet b1, and a switching outlet b2; the third switching valve 5 is provided with a sample inlet c1, a switching inlet c1, and a switching outlet c1; the fourth switching valve 2 is provided with a sample inlet d1, a switching inlet d1, and a switching outlet d1; an outlet of the first pump body 15 and an outlet of the second pump body 16 both communicate with the switching inlet a1 of the first switching valve 6; the switching outlet a1 of the first switching valve 6 communicates with the switching inlet b1 of the second switching valve 10; the switching outlet b1 of the second switching valve 10 communicates with an inlet of the synthetic column 9; an outlet of the synthetic column 9 communicates with the switching inlet c1 of the third switching valve 5; the switching outlet c1 of the third switching valve 5 communicates with an inlet of the first pump body 15; the switching outlet a2 of the first switching valve 6 communicates with an inlet of the activation reactor 8; an outlet of the activation reactor 8 communicates with the switching inlet b2 of the second switching valve 10; the switching outlet b2 of the second switching valve 10 communicates with the switching inlet d1 of the fourth switching valve 2; and the switching outlet d1 of the fourth switching valve 2 communicates with an inlet of the second pump body 16.

By using the solid-phase peptide synthesis apparatus provided in this embodiment, the first circulation pipeline 100, the second circulation pipeline 200, the first switching valve 6, and the second switching valve 10 can constitute a deprotection circulation, an amino acid activation circulation, and an amino acid coupling circulation according to their communication relationships. In the deprotection circulation, a deprotection reagent enters the third switching valve 5 via the sample inlet c1, is conveyed to the first switching valve 6 via the first pump body 15, and then enters the synthetic column 9 via the second switching valve 10; and at the moment, a circulation pipeline of synthetic column 9→third switching valve 5→first pump body 15→first switching valve 6→second switching valve 10→synthetic column 9 is opened to cause the deprotection reagent to be in full contact with a carrier in the synthetic column 9. In the amino acid activation circulation, amino acids and a reagent are combined into a single stream via the first pump body 15 and the second pump body 16, enter the first switching valve 6, and then enter the activation reactor 8; and then, a circulation pipeline of activation reactor 8→second switching valve 10→fourth switching valve 2→second pump body 16→first switching valve 6→activation reactor 8 is opened to cause the amino acids to be in full contact with an activating agent, thereby achieving an effect of activating the amino acids. In the amino acid coupling circulation, the amino acids are fully activated and then enter the synthetic column 9; and then, a circulation pipeline of synthetic column 9→third switching valve 5→first pump body 15→first switching valve 6→second switching valve 10→synthetic column 9 is opened to cause the activated amino acids to be in full contact with the carrier. By using the above structures, continuous fully-automatic synthesis can be realized, synthesis continuity is ensured maximally, synthesis intelligence is also realized, operation time of each process and between processes is shortened, production efficiency may be improved, and the amount of the amino acids used may be reduced, thereby reducing costs.

It is to be noted that, the second switching valve 10 is a column switching valve, which provides three pathway functions to meet functions that allow materials to circulate and pass through. The activation reactor 8 is an online activation reactor with a mixed core, and the synthetic column 9 is a peptide synthetic column.

As shown in Fig. 1, in this embodiment, the first circulation pipeline 100 further includes a fifth switching valve 7; the fifth switching valve 7 is provided with a switching inlet e1, a first flushing inlet, and a switching outlet e1; the outlet of the synthetic column 9 communicates with the switching inlet e1 of the fifth switching valve 7; the switching outlet e1 of the fifth switching valve 7 communicates with the switching inlet c1 of the third switching valve 5; and the switching outlet b2 of the second switching valve 10 also communicates with the first flushing inlet of the fifth switching valve 7.

When the solid-phase peptide synthesis apparatus needs to be flushed, flushing may be realized by opening the following valves: third switching valve 5→first pump body 15→first switching valve 6→activation reactor 8→second switching valve 10→fifth switching valve 7.

In this embodiment, the fifth switching valve 7 is further provided with a second flushing inlet; the first switching valve 6 is further provided with a switching outlet a3; and the switching outlet a3 of the first switching valve 6 communicates with the second flushing inlet of the fifth switching valve 7.

When the solid-phase peptide synthesis apparatus starts to be used, a reagent needs to be pre-injected in the pipeline to cause the reagent to be filled in the pipeline, and the reagent preferably does not pass through components other than the valves, such that the above objective may be implemented by the following valves: third switching valve 5→first pump body 15→first switching valve 6→fifth switching valve 7.

As shown in Fig. 1, the solid-phase peptide synthesis apparatus further includes a waste liquid pipeline 300. The third switching valve 5 is further provided with a waste liquid outlet, and the waste liquid outlet of the third switching valve 5 communicates with an inlet of the waste liquid pipeline 300. Waste liquid is collected by using the waste liquid pipeline, thereby improving an environmental protection effect of the apparatus.

Specifically, a flow restrictor 13 and a waste liquid outlet valve 14 are arranged on the waste liquid pipeline 300; and the waste liquid outlet valve 14 is located downstream of the flow restrictor 13. The flow restrictor 13 may ensure the accuracy of a pump by transmitting an electrical signal to a controller. The waste liquid outlet valve 14 performs collection by classifying the waste liquid into 8 types, and recycling and reuse may be achieved, such that an energy-saving effect can be achieved.

It is to be noted that, the first switching valve 6 is a three-point selection valve, which may select a pathway of activation reactor 8-second pump body 16 for circulation, or a pathway of synthetic column 9-first pump body 15 for circulation, or a pathway of fifth switching valve 7-waste liquid outlet valve 14.

In this embodiment, the solid-phase peptide synthesis apparatus further includes a detector that is capable of detecting a synthesis parameter. The detector is disposed on the first circulation pipeline 100 to detect a synthesis effect.

Specifically, the detector includes a UV detector 11, and the UV detector 11 is located between the synthetic column 9 and the third switching valve 5. The UV detector 11 may detect UV absorption online during synthesis.

The detector further includes a conductivity detector 3, and the conductivity detector 3 is located between the first pump body 15 and the first switching valve 6. The conductivity detector 3 may test a conductance signal online during synthesis.

Specifically, the detector further comprises a pressure detector (which detects a system pressure during synthesis) and a temperature detector (which detects a system temperature during synthesis), so as to further improve detection accuracy and precision.

In this embodiment, the solid-phase peptide synthesis apparatus further includes a controller. The first pump body 15, the second pump body 16, and the detector are respectively in signal connection with the controller, achieving the automatic control operation of the apparatus, thereby improving production efficiency.

A serial port server is connected with a host via an Ethernet port, and the serial port server is connected with the plurality of pumps and the plurality of detectors, respectively. Specifically, the controller includes a microcontroller unit, a communication module, a storage module, a plurality of communication interfaces connected with the microcontroller unit, and a sample injection valve module, a valve power supply control module, a mixed component control module, a plurality of detection modules, and a pressure collection module, which are respectively connected with the microcontroller unit. The microcontroller unit is connected with the serial port server through the communication module, thereby achieving signal interaction with the host.

As shown in Fig. 1, the solid-phase peptide synthesis apparatus further includes two first sample injection valves 1, and outlets of the two first sample injection valves 1 both communicate with the sample inlet d1 of the fourth switching valve 2, so as to achieve amino acid sample injection. Specifically, the first sample injection valve 1 may provide inlets for 13 types of amino acids.

As shown in Fig. 1, the solid-phase peptide synthesis apparatus further includes a second sample injection valve 4, and an outlet of the second sample injection valve 4 communicates with the sample inlet c1 of the third switching valve 5, so as to achieve reagent sample injection. Specifically, the second sample injection valve 4 may provide inlets for 6 types of reagents and an inlet for flushing.

It is to be noted that, the third switching valve 5 is a two-point selection valve, which may select a pathway of second sample injection valve 4-first pump body 15 or a pathway of UV detector 11-first pump body 15 for coupling circulation.

A pipe body of the first circulation pipeline 100 is made of a PEEK, FEP, or stainless steel material; and a pipe body of the second circulation pipeline 200 is made of the PEEK, FEP, or stainless steel material. Therefore, the pipe body of the first circulation pipeline 100 and the pipe body of the second circulation pipeline 200 both have the advantages of low costs and long service life.

In this embodiment, the pipe body of the first circulation pipeline 100 and the pipe body of the second circulation pipeline 200 use the PEEK or FEP, with a pipe diameter being 1/8 and 1/16 inches.

It is to be noted that, all the valve bodies in this embodiment are multi-channel control valves, a perform operation of each valve controls on and off by executing the rotation of a motor driver, drives a rotor fixed on a shaft end to rotate, and switches to a channel of a required valve head, thereby controlling the on and off of the pipeline connected with the single valve body. A connection manner of the valve body includes threaded or ferrule connection, chuck connection, flange connection, etc. In other embodiments, the number of sample injection valves may be increased, and a valve control manner is changed, for example, an electric valve or a pneumatic valve. Alternatively, a feeding requirement may be met by increasing the number of pumps, and the type of pumps is changed, for example, an electric or pneumatic diaphragm pump, a plunger pump, a mechanical pump, a magnetic drive pump, etc.

In this embodiment, the inventor uses the solid-phase peptide synthesis apparatus to perform a test, and a synthetic amino acid sequence in this test is H-Val-GIn-Ala-Ala-Ile-Asp-Tyr-Ile-Asn-Gly-NH2.

A test process includes: (1) device debugging: pre-feeding is performed, and it is confirmed that a device may operates normally; (2) preparation: material mixing and column filling are performed; (3) synthesis: automatic feeding is performed for peptide synthesis according to a set synthesis sequence (deprotection, washing, activation, washing, synthesis, washing, capping, washing, the amino acids are sequentially coupled according to the sequence, until a set length of a target chain is achieved); and (4) cutting: a complete peptide chain is detached from a carrier.

The above describes general steps for testing using the solid-phase peptide synthesis apparatus. After cutting is completed, product post-processing and purity detection also need to be performed. According to detection results, it may be learned that the purity of a peptide chain synthesized using the solid-phase peptide synthesis apparatus of this embodiment is 91.32%,
which is superior to purity (approximately 87.56%) in the related art.

Through the apparatus provided in this embodiment, the beneficial effects are as follows.
(1) Optimization is performed for problems in existing peptide synthesis devices, synthesis continuity is ensured maximally in a manner of continuous flow synthesis, and operation time of each process and between processes is shortened, thereby greatly improving production efficiency.
(2) Existing peptide synthesis devices are large in size and unsuitable for research and development experiments, such that optimization is performed. By using a rational pipeline layout (on the basis of the overall layout, pipelines that require materials to pass through and generate residues are optimized and shortened), devices and pipelines are simple in structure and small in dead volume, material residues are reduced, the risk of cross-contamination between materials is reduced, and the effect of reducing the materials is achieved.
(3) Products are easily damaged due to the mechanical stirring of existing peptide synthesis devices, such that optimization is performed. Compared to a material circulation manner used by a traditional synthesis reactor, the solid-phase peptide synthesis apparatus replaces a stirring apparatus, reducing a shearing force exerted on resin by mechanical stirring, and minimizing the risk of product damage, thereby increasing yields.
(4) Through a unique pipeline design, various circulation modes may be realized without manual switching, such that synthesis efficiency is improved while costs are reduced.
(5) For the problem of lack of process monitoring instruments in existing peptide synthesis devices, real-time monitoring is performed on partial synthesis process by introducing the pressure detector, a temperature detector, the UV detector 11, and the conductivity detector 3, such that partial synthesis process may be determined online to guide correction during synthesis, thereby improving product purity.

The above are only the preferred embodiments of the present disclosure and are not intended to limit the present disclosure. For those skilled in the art, the present disclosure may have various modifications and variations. Any modifications, equivalent replacements, improvements and the like made within the spirit and principle of the present disclosure shall fall within the scope of protection of the present disclosure.

## Claims

1. A solid-phase peptide synthesis apparatus, comprising a first circulation pipeline (100), a second circulation pipeline (200), a first switching valve (6), and a second switching valve (10), wherein the first circulation pipeline (100) comprises a synthetic column (9), a third switching valve (5), and a first pump body (15), which are sequentially arranged from upstream to downstream; the second circulation pipeline (200) comprises a fourth switching valve (2), a second pump body (16), and an activation reactor (8), which are sequentially arranged from upstream to downstream;
the first switching valve (6) is provided with a switching inlet a1, a switching outlet a1, and a switching outlet a2; the second switching valve (10) comprises a switching inlet b1, a switching inlet b2, a switching outlet b1, and a switching outlet b2; the third switching valve (5) is provided with a sample inlet c1, a switching inlet c1, and a switching outlet c1; the fourth switching valve (2) is provided with a sample inlet d1, a switching inlet d1, and a switching outlet d1; an outlet of the first pump body (15) and an outlet of the second pump body (16) both communicate with the switching inlet a1 of the first switching valve (6); the switching outlet a1 of the first switching valve (6) communicates with the switching inlet b1 of the second switching valve (10); the switching outlet b1 of the second switching valve (10) communicates with an inlet of the synthetic column (9); an outlet of the synthetic column (9) communicates with the switching inlet c1 of the third switching valve (5); the switching outlet c1 of the third switching valve (5) communicates with an inlet of the first pump body (15); the switching outlet a2 of the first switching valve (6) communicates with an inlet of the activation reactor (8); an outlet of the activation reactor (8) communicates with the switching inlet b2 of the second switching valve (10); the switching outlet b2 of the second switching valve (10) communicates with the switching inlet d1 of the fourth switching valve (2); and the switching outlet d1 of the fourth switching valve (2) communicates with an inlet of the second pump body (16).

2. The solid-phase peptide synthesis apparatus according to claim 1, wherein the first circulation pipeline (100) further comprises a fifth switching valve (7); the fifth switching valve (7) is provided with a switching inlet e1, a first flushing inlet, and a switching outlet e1; the outlet of the synthetic column (9) communicates with the switching inlet e1 of the fifth switching valve (7); the switching outlet e1 of the fifth switching valve (7) communicates with the switching inlet c1 of the third switching valve (5); and the switching outlet b2 of the second switching valve (10) also communicates with the first flushing inlet of the fifth switching valve (7).

3. The solid-phase peptide synthesis apparatus according to claim 2, wherein the fifth switching valve (7) is further provided with a second flushing inlet; the first switching valve (6) is further provided with a switching outlet a3; and the switching outlet a3 of the first switching valve (6) communicates with the second flushing inlet of the fifth switching valve (7).

4. The solid-phase peptide synthesis apparatus according to claim 1, further comprising a waste liquid pipeline (300), wherein the third switching valve (5) is further provided with a waste liquid outlet, and the waste liquid outlet of the third switching valve (5) communicates with an inlet of the waste liquid pipeline (300).

5. The solid-phase peptide synthesis apparatus according to claim 4, wherein a flow restrictor (13) and a waste liquid outlet valve (14) are arranged on the waste liquid pipeline (300); and the waste liquid outlet valve (14) is located downstream of the flow restrictor (13).

6. The solid-phase peptide synthesis apparatus according to claim 1, further comprising a detector that is capable of detecting a synthesis parameter, wherein the detector is disposed on the first circulation pipeline (100).

7. The solid-phase peptide synthesis apparatus according to claim 6, wherein
the detector comprises a UV detector (11), and the UV detector (11) is located between the synthetic column (9) and the third switching valve (5); and/or
the detector further comprises a conductivity detector (3), and the conductivity detector (3) is located between the first pump body (15) and the first switching valve (6).

8. The solid-phase peptide synthesis apparatus according to claim 6, further comprising a controller, wherein the first pump body (15), the second pump body (16), and the detector are respectively in signal connection with the controller.

9. The solid-phase peptide synthesis apparatus according to any one of claims 1 to 8,
the solid-phase peptide synthesis apparatus further comprises two first sample injection valves (1), wherein outlets of the two first sample injection valves (1) both communicate with the sample inlet d1 of the fourth switching valve (2); and/or
the solid-phase peptide synthesis apparatus further comprises a second sample injection valve (4), wherein an outlet of the second sample injection valve (4) communicates with the sample inlet c1 of the third switching valve (5).

10. The solid-phase peptide synthesis apparatus according to any one of claims 1 to 8, wherein
a pipe body of the first circulation pipeline (100) is made of a Poly Ether Ether Ketone (PEEK), Fluorinated Ethylene Propylene (FEP), or stainless steel material; and/or
a pipe body of the second circulation pipeline (200) is made of the PEEK, FEP, or stainless steel material.
